# EUROPÄISCHE PATENTSCHRIFT

(11) **EP 2 297 292 B1**
(45) Veröffentlichungstag und Bekanntmachung des Hinweises auf die Patenterteilung: **22.04.2020**
(21) Anmeldenummer: 09757153.3
(22) Anmeldetag: 07.05.2009
(51) Int. Cl.: C12M 1/04

(54) **BIOREAKTOR MIT KONDENSATOR**
BIOREACTOR HAVING CONDENSER
BIORÉACTEUR À CONDENSATEUR

(30) Priorität: 30.05.2008 DE 102008025968
(43) Veröffentlichungstag der Anmeldung: 23.03.2011
(73) Patentinhaber: Sartorius Stedim Biotech GmbH, 37079 Göttingen (DE)
(72) Erfinder: REIF, Oscar-Werner, 30173 Hannover (DE); VAN DEN BOOGAARD, Jürgen, 37127 Dransfeld (DE); HELLWIG, Gerid, 37083 Göttingen (DE); NOACK, Ute, 37079 Göttingen (DE)
(86) Internationale Anmeldenummer: PCT/EP2009/003255
(87) Internationale Veröffentlichungsnummer: WO 2009/146769

(56) Entgegenhaltungen:
- DE-A1- 1 601 144
- GB-A- 449 621
- US-A- 5 443 985
- US-A- 5 958 763
- US-A- 6 133 021
- US-A1- 2005 272 146
- US-A1- 2006 033 222

## Beschreibung

### Gebiet der Erfindung

Die Erfindung betrifft einen Bioreaktor mit einem Behälter mit mindestens einem Gasabführungskanal zum Gasaustrag, wobei die Öffnung des Gasabführungskanals mit einem Sterilfilter und einem dazwischen angeordneten Kondensator mit Kondensationsflächen verbunden ist.

Die Erfindung betrifft weiterhin einen Kondensator für einen Bioreaktor.

### Stand der Technik

Beim Begasen eines Bioreaktors nimmt die zugeführte Luft beim Durchströmen des Mediums Feuchte auf. Der gleiche Effekt tritt ein bei biotechnologischen Prozessen, in denen Gase als Stoffwechselprodukte von Mikroorganismen gebildet und aus dem Bioreaktor abgeführt werden. Durch den Einsatz eines Abflusskondensators im Abflusssystem kann man verhindern, dass die Abluft einen Teil des Mediums mit hinausträgt. Warme, feuchte Abluft wird durch den Kondensator soweit heruntergekühlt, dass das in der Abluft enthaltene Medium als Kondensat in den Kessel zurückfließt.

Aus der US 5 443 985 A ist ein Biorektor mit einem Behälter aus einem Bor-Silikat-Glas oder rostfreiem Stahl bekannt, der an seinem in vertikaler Richtung oberen Ende einen Gasabführungskanal zum Gasaustrag aufweist. Dabei ist die Öffnung des Gasabführungskanals mit einem Sterilfilter verschlossen. Zwischen dem Behälter und dem Sterilfilter ist ein wassergekühlter Kondensator angeordnet, wobei Teile der Innenflächen des Gasabführungskanals die bei einem Kondensator benötigten Kondensationsflächen bilden. Dem Behälter des bekannten Bioreaktors wird an seinem in vertikaler Richtung unterem Ende über einen Gasführungskanal Gas zugeführt.

Bei dem bekannten Bioreaktor bildet die den Gasabführungskanal umschließende Innenwandung die Kondensationsfläche des Kondensators. Der Kondensator weist eine doppelte Wandung auf, durch die Kühlwasser hindurchgeführt wird.

Nachteilig dabei ist, dass bei weitgehend laminarer Strömung ein relativ langer Gasabführungskanal benötigt wird, um den gesamten Abluftstrom ausreichend abzukühlen. Insbesondere bei Verwendung eines Bioreaktors als Einweg-Reaktor mit einem Kondensator ist es schwierig, eine kompakte Bauform zu erreichen. Besonders kritisch erweist sich dabei das mögliche Verblocken des hydrophoben Sterilfilters, wenn der Behälter des Bioreaktors als flexibler Kunststoffbeutel ausgebildet ist, dessen Innendruck bestimmte Grenzwerte nicht überschreiten darf, so dass gegebenenfalls die Begasung und damit der Fermentationsprozess abgebrochen werden muss.

Weiterhin ist aus der US 6 133 021 A ein Bioreaktor bekannt, dessen Gasabführungskanal mit einem Kondensator verbunden ist. Auch dieser Kondensator weist die oben beschriebenen Nachteile auf.

Weiterhin ist aus der DD 260 837 A3 ein Bioreaktor mit einem Behälter bekannt, der einen Gasabführungskanal zum Gasaustrag aufweist. Die Öffnung des Gasabführungskanals ist mit einem Abluftfilter verbunden, wobei zwischen Behälter und Abluftfilter ein Kondensator angeordnet ist.

Auch dieser bekannte Bioreaktor weist die oben genannten Nachteile auf.

Aus der EP 1 167 905 A2 ist eine Vorrichtung zur Entfernung von flüchtigen Komponenten aus einem Gasstrom unter Verwendung eines kryogenen Prozesses bekannt. Um die flüchtigen Komponenten aus einem Prozessgasstrom zu entfernen, wird der Gasstrom in einem Kondensator gekühlt, um die flüchtigen Komponenten in Flüssigkeit und Eis umzuformen. Das von den flüchtigen Komponenten befreite Prozessgas, das noch Eispartikel von flüchtigen Komponenten enthalten kann, passiert einen Filter, der stromabwärts von dem Kondensator angeordnet ist, um die Eispartikel zu entfernen, die eine Größe von mehr als 50 µm aufweisen.

Diese technische Anwendung ist mit ihrem Kälteprozess für Bioreaktoren nicht geeignet bzw. nicht übertragbar. Der in der EP 1 167 905 A2 verwendete Wirbel von flüssigem Kryogen zur Kühlung ist zum einen für Bioreaktoren ungeeignet und zum anderen wird er verwendet, um größere Eiskristalle zu erzeugen.

Aus der GB 449 621 A ist eine Vorrichtung zur Rückgewinnung von Phthalsäureanhydrid (PSA) aus Dämpfen bekannt, bei der die Dämpfe als ein turbulenter Strom durch einen rohrförmigen Kondensator geführt werden. Der Fachmann erhält keinen Hinweis für die Entwicklung von Bioreaktoren.

Aus der US 3 063 259 A ist eine Vorrichtung zum Filtern und Dehydrieren von Gasen bekannt. Bei dem aus der US 3 063 259 A bekannten Kondensor wird das Prozessgas spiralförmig um eine spiralförmig ausgebildete Kühlwandung geleitet. Auf eine turbulente Strömung im Gasabführungskanal kommt es dabei nicht an. Auch hierbei erhält der Fachmann keinen Hinweis für die Entwicklung von Bioreaktoren
Aus der WO 2006/020177 A1 ist ein Photobioreaktor für photosynthetische Organismen, wie Algen, bekannt. Dabei lehrt die WO 2006/020177 A1, dass in bestimmten Ausführungsformen zur Vermeidung des Austritts von Algen aus dem Gasauslass des Bioreaktors ein Filter, wie beispielsweise ein hydrophober Filter mit einem Hauptporendurchmesser kleiner als der mittlere Durchmesser der Algen, verwendet werden kann. Derartige Algenfilter sind keine Sterilfilter.

US 5 958 763 beschreibt zum Ausleiten von Gas aus einem Bioreaktor ein gekoppeltes Medien-und Gasstromsystem, bei dem das abzuführende Gas über den Auslass über einen Kondensator und einen hydrophoben Filter in einen Medienbehälter geleitet wird. Im Bereich des Kondensators befinden sich sog. Turbulenzerzeugungsmittel in Form von Glas- oder Kunststoffkügelchen.

### Aufgabenstellung

Aufgabe der vorliegenden Erfindung ist es daher, die bekannten Bioreaktoren und ihre Kondensatoren so zu verbessern, dass sie bei kompakter Bauform aus Kunststoff, insbesondere zur Einmal-Verwendung, ausgebildet werden können.

### Darstellung der Erfindung

Die Aufgabe wird in Verbindung mit den Merkmalen des Anspruches 1 dadurch gelöst, dass der Sterilfilter als ein hydrophober Filter ausgebildet ist, dass im Bereich des Kondensators im Gasabführungskanal eine turbulente Strömung erzeugende Turbulenzerzeugungsmittel angeordnet sind, und dass der Behälter, der Kondensator und der Sterilfilter aus gammasterilisierbarem Kunststoff ausgebildet sind.

Durch die Anordnung von Turbulenzerzeugungsmitteln im Gasabführungskanal, wird sichergestellt, dass das abgeführte Gas bzw. die Abluft an den Kondensationsflächen des Kondensators vorbeigeführt wird. Dies ermöglicht einen effizienteren Wärmeaustausch zwischen dem abgeführten Gas und dem Kühlmittel des Kondensators. Der effizientere Wärmeaustausch ermöglicht trotz Verwendung von Kunststoff eine kompaktere Bauweise. Auch die Ausbildung des Sterilfilters als hydrophoben Sterilfilter unterstützt eine kompakte Bauweise. Die Ausbildung des Behälters, des Kondensators und des Sterilfilters aus gammasterilisierbarem Kunststoff ist besonders vorteilhaft für eine Lieferung gebrauchsfertig vorsterilisierter Bioreaktorsysteme an Kunden.

Gemäß einer bevorzugten Ausführungsform der Erfindung sind die Außenflächen der Turbulenzerzeugungsmittel als Kondensationsflächen ausgebildet. Beispielsweise kann das Turbulenzerzeugungsmittel als ein wendelförmig im Gasabführungskanal angeordneter Kondensatorschlauch oder Kondensatorrohr ausgebildet sein, durch die eine Kühlflüssigkeit leitbar ist.

Nach einer weiteren bevorzugten Ausführungsform der Erfindung bildet die den Gasabführungskanal umschließende Innenwandung die Kondensationsfläche, wobei in einem Abstand zur Innenwandung eine Außenwandung angeordnet ist und durch den Zwischenraum zwischen Außenwandung und Innenwandung eine Kühlflüssigkeit durchgeleitet wird. Das Turbulenzerzeugungsmittel ist dabei als eine Packung ausgebildet, die beispielsweise aus einem Vliesmaterial besteht.

Nach einer weiteren Ausführungsform der Erfindung ist die Packung aus Rohrabschnitten, so genannten Raschig Ringen ausgebildet. Die Rohrabschnitte können dabei aus Glas, Keramik, Kunststoff oder Metall ausgebildet sein.

Gemäß einer weiteren bevorzugten Ausführungsform der Erfindung weist der Sterilfilter an seinem Umfang eine Heizung auf, die es ermöglicht, die Abluft zu temperieren, wodurch ein Verblocken der hydrophoben Filter durch kondensierte Flüssigkeiten vermieden wird.

Gemäß einer weiteren bevorzugten Ausführungsform der Erfindung ist der Sterilfilter in den Kondensator bzw. dessen Gehäuse integriert. Dies ermöglicht eine besonders kompakte Bauweise.

Der gesamte Bioreaktor, bestehend aus dem Behälter, dem Kondensator und dem Sterilfilter, ist nach einer weiteren Ausführungsform als eine Einwegvorrichtung ausgebildet. Vorzugsweise ist dabei der Behälter als ein flexibler Beutel ausgebildet.

Die Aufgabe bezüglich eines Kondensators wird in Verbindung mit den Merkmalen des Anspruches 13 weiterhin dadurch gelöst, dass im Bereich des Kondensators im Gasabführungskanal eine turbulente Strömung erzeugende Turbulenzerzeugungsmittel angeordnet sind.

Der Kondensator weist die oben geschilderten Vorteile auf. Gemäß einer bevorzugten Ausführungsform der Erfindung ist das Turbulenzerzeugungsmittel als ein wendelförmig im Gasabführungskanal angeordneter Kondensatorschlauch oder Kondensatorrohr ausgebildet, durch die eine Kühlflüssigkeit leitbar ist.

In einer weiteren bevorzugten Ausführungsform der Erfindung ist der Kondensatorschlauch von weiteren kombinierten Kühl- und Turbulenzerzeugungsmitteln umgeben, wie Vliesmaterial oder geformten dünnen Einzelstücken, vorzugsweise aus Kunststoff. Diese Mittel wirken neben der Turbulenzerzeugung aufgrund ihrer Lage oder ihres Kontaktes mit dem Kondensatorschlauch als Kühlflächen und leiten Wärme über Wärmebrücken ab.

Nach einer weiteren bevorzugten Ausführungsform der Erfindung bildet die den Gasabführungskanal umschließende Innenwandung die Kondensationsfläche, wobei in einem Abstand zur Innenwandung eine Außenwandung angeordnet ist und durch den Zwischenraum zwischen Außenwandung und Innenwandung eine Kühlflüssigkeit durchleitbar ist.

Das Turbulenzerzeugungsmittel kann dabei als eine Packung aus einem Vliesmaterial oder aus Rohrabschnitten aus Glas, Keramik, Kunststoff oder Metall ausgebildet sein.

Weitere Merkmale der Erfindung ergeben sich aus der nachfolgenden ausführlichen Beschreibung und den beigefügten Zeichnungen, in denen bevorzugte Ausführungsformen der Erfindung beispielhaft veranschaulicht sind.

In den Zeichnungen zeigen:
- Figur 1:: eine Seitenansicht eines Bioreaktors mit Behälter, Kondensator und Sterilfilter im Schnitt und Ausriss,
- Figur 2:: eine Seitenansicht im Schnitt eines Kondensators mit integriertem Sterilfilter und
- Figur 3:: eine Seitenansicht im Schnitt eines Kondensators.

Ein Bioreaktor 1 besteht im Wesentlichen aus einem Behälter 2, einem Gasabführungskanal 3, einem Kondensator 4, einem Sterilfilter 5 und einem Gaszuführungskanal 6.

Der Behälter 2 ist als ein Beutel aus Kunststoff mit einer flexiblen Wandung 7 ausgebildet und weist u.a. neben dem Gasabführungskanal 3 den Gaszuführungskanal 6 auf.

Die Öffnung 8 des Gasabführungskanals 3 ist mit dem hydrophoben Sterilfilter 5 verbunden. Zwischen Behälter 2 und dem Sterilfilter 5 ist in dem Gasabführungskanal 3 der Kondensator 4 angeordnet.

Im Bereich des Kondensators 4 sind im Gasabführungskanal 3 zur Erzeugung einer turbulenten Strömung Turbulenzerzeugungsmittel 9 angeordnet.

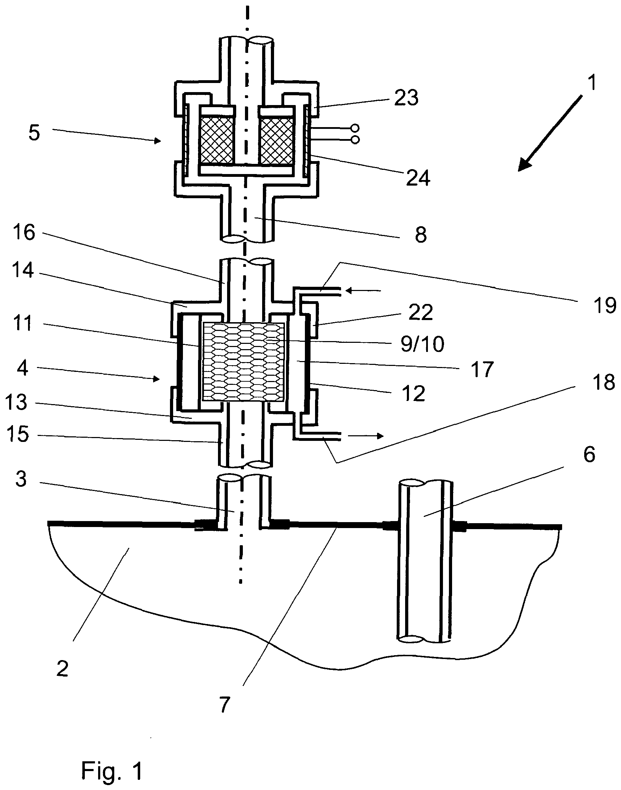

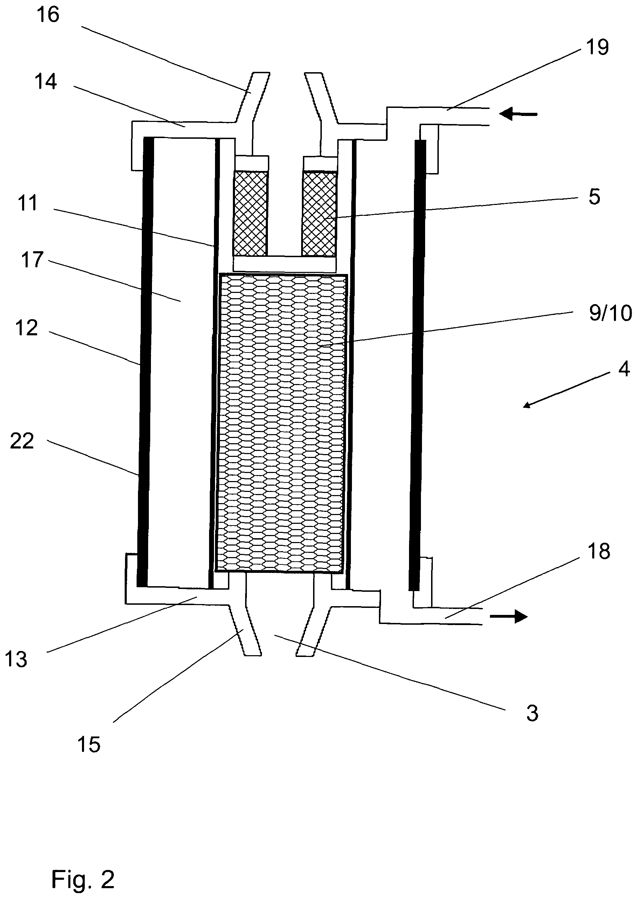

Entsprechend dem Ausführungsbeispiel von Figur 1 und Figur 2 sind die Turbulenzerzeugungsmittel 9 als eine in den Gasabführungskanal 3 eingesteckte Packung 10 aus einem Vliesmaterial oder aus Rohrabschnitten aus Glas, Keramik, Kunststoff oder Metall ausgebildet, und es wird die den Gasabführungskanal 3 umschließende Wandung 11 von einem Innenrohr aus Kunststoff, bevorzugt aus Polycarbonat, Polyethylen oder Polypropylen gebildet. In einem Abstand zur Innenwandung 11 weist der Kondensator 4 eine Außenwandung 12 auf, die von einem Außenrohr aus Polypropylen gebildet wird. An seinen Stirnseiten weist der Kondensator 4 jeweils einen Deckel 13, 14 mit einem Anschluss 15, 16 für den Gasabführungskanal 3 auf. In den von den Wandungen 11, 12 gebildeten Zwischenraum 17 werden über einen unteren Kühlmittelanschluss 18 Kühlmittel ab- und über einen oberen Kühlmittelanschluss 19 zugeführt.

Die Kondensationsfläche des Kondensators 4 wird entsprechend den Ausführungsbeispielen von Figur 1 und 2 durch die Innenwandung 11 gebildet.

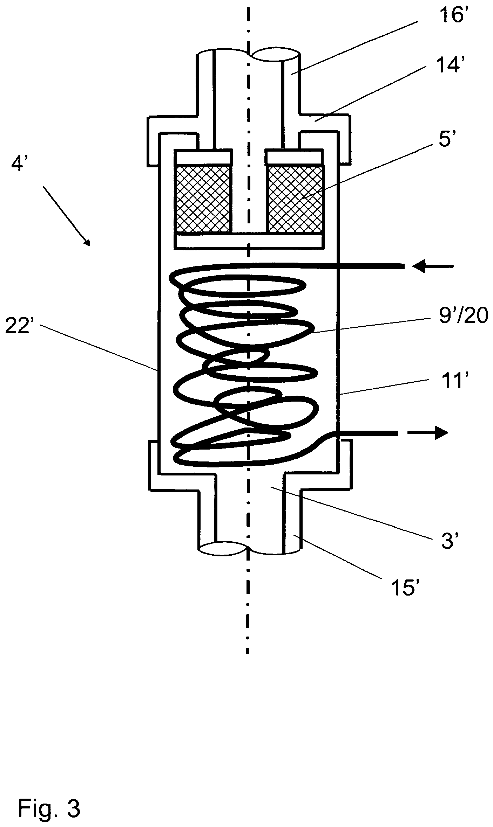

Entsprechend dem Ausführungsbeispiel von Figur 3 weist der Kondensator 4' eine Innenwandung 11' des Gasabführungskanals 3' auf, die von einem Rohr aus Kunststoff gebildet wird, das gleichzeitig die Außenwandung bildet. Das in dem Gasabführungskanal 3' angeordnete Turbulenzerzeugungsmittel 9' wird von einem wendelförmig angeordneten Kondensatorschlauch 20 gebildet, durch den über seinen unteren Anschluss 15' Kühlmittel ab- und über seinen oberen Anschluss 16' Kühlmittel zugeführt wird. Dabei bildet die Außenfläche 21 dessen Kondensatorschlauches 20 die Kondensationsfläche des Kondensators 4'. Der Kondensatorschlauch 20 kann ebenfalls aus Kunststoff ausgebildet sein.

Der Kondensatorschlauch 20 kann von weiteren - nicht dargestellten - kombinierten Kühl- und Turbulenzerzeugungsmitteln 9 umgeben sein, wie Vliesmaterial oder geformten dünnen Einzelstücken aus vorzugsweise Kunststoff.

Gemäß den Ausführungsbeispielen der Figuren 2 und 3 ist der Sterilfilter 5 in das Gehäuse 22, 22' des Kondensators 4, 4' integriert. Der Sterilfilter 5, 5' ist dabei jeweils mit dem oberen Deckel 14, 14' verschweißt.

Entsprechend dem Ausführungsbeispiel von Figur 1 weist der hydrophobe Sterilfilter 5 an seinem Umfang bzw. Gehäuse 23 eine Heizung 24 zur Temperierung des abzuführenden Gases auf.

## Patentansprüche

1. Bioreaktor (1) mit einem Behälter (2) mit mindestens einem Gasabführungskanal (3, 3') zum Gasaustrag, wobei die Öffnung (8) des Gasabführungskanals (3, 3') mit einem hydrophoben Sterilfilter (5, 5') und einem dazwischen angeordneten Kondensator (4, 4') mit Kondensationsflächen verbunden ist,
**dadurch gekennzeichnet,**
**dass** im Bereich des Kondensators (4, 4') im Gasabführungskanal (3, 3') eine turbulente Strömung erzeugende Turbulenzerzeugungsmittel (9, 9') angeordnet sind, und
**dass** der Behälter (2), der Kondensator (4, 4') und der Sterilfilter (5, 5') aus gammasterilisierbarem Kunststoff ausgebildet sind.

2. Bioreaktor, nach Anspruch 1,
**dadurch gekennzeichnet,**
**dass** die Außenflächen der Turbulenzerzeugungsmittel (9, 9') als Kondensationsflächen ausgebildet sind.

3. Bioreaktor nach Anspruch 2,
**dadurch gekennzeichnet,**
**dass** das Turbulenzerzeugungsmittel (9, 9') als ein wendelförmig im Gasabführungskanal (3, 3') angeordneter Kondensatorschlauch (20) oder Kondensatorrohr ausgebildet ist, durch die eine Kühlflüssigkeit leitbar ist.

4. Bioreaktor nach Anspruch 3,
**dadurch gekennzeichnet,**
**dass** das der Kondensatorschlauch (20) oder das Kondensatorrohr von weiteren kombinierten Kühl- und Turbulenzerzeugungsmitteln umgeben sind.

5. Bioreaktor nach Anspruch 1,
**dadurch gekennzeichnet,**
**dass** die den Gasabführungskanal (3, 3') umschließende Innenwandung (11, 11') die Kondensationsfläche bildet.

6. Bioreaktor nach Anspruch 5,
**dadurch gekennzeichnet,**
**dass** in einem Abstand zur Innenwandung (11, 11') eine Außenwandung (12) angeordnet ist und
**dass** durch den Zwischenraum (17) zwischen Außenwandung (12) und Innenwandung (11, 11') eine Kühlflüssigkeit durchleitbar ist.

7. Bioreaktor nach einem der Ansprüche 1, 5 oder 6,
**dadurch gekennzeichnet,**
**dass** das Turbulenzerzeugungsmittel (9, 9') als eine Packung (10) ausgebildet ist.

8. Bioreaktor nach Anspruch 7,
**dadurch gekennzeichnet,**
**dass** die Packung (10) aus einem Vliesmaterial ausgebildet ist.

9. Bioreaktor nach Anspruch 7,
**dadurch gekennzeichnet,**
**dass** die Packung (10) aus Rohrabschnitten aus Glas, Keramik, Kunststoff oder Metall ausgebildet ist.

10. Bioreaktor nach einem der Ansprüche 1 bis 9,
**dadurch gekennzeichnet,**
**dass** der Sterilfilter (5, 5') an seinem Umfang eine Heizung (24) aufweist.

11. Bioreaktor nach einem der Ansprüche 1 bis 10,
**dadurch gekennzeichnet,**
**dass** der Sterilfilter (5, 5') in den Kondensator (4, 4') integriert ist.

12. Bioreaktor nach einem der Ansprüche 1 bis 11,
**dadurch gekennzeichnet,**
**dass** der Behälter (2), der Kondensator (4, 4') und der Sterilfilter (5, 5') als eine Einwegvorrichtung ausgebildet sind.

13. Kondensator (4, 4') für einen Bioreaktor (1) gemäß einem der Ansprüche 1 bis 12, mit einem Gehäuse (22, 22') mit einem Gasabführungskanal (3, 3') zur Durchleitung eines Gases und Kondensierung an Kondensatflächen,
**dadurch gekennzeichnet,**
**dass** im Bereich des Kondensators (4, 4') im Gasabführungskanal (3, 3') mit hydrophoben Sterilfilter (5, 5') eine turbulente Strömung erzeugende Turbulenzerzeugungsmittel (9, 9') angeordnet sind und der Kondensator (4, 4') als auch der Sterilfilter (5, 5') aus gammasterilisierbarem Kunststoff ausgebildet sind.

14. Kondensator nach Anspruch 13,
**dadurch gekennzeichnet,**
**dass** das Turbulenzerzeugungsmittel (9, 9') als ein wendelförmig im Gasabführungskanal (3, 3') angeordneter Kondensatorschlauch (20) oder Kondensatorrohr ausgebildet sind, durch die eine Kühlflüssigkeit leitbar ist.

15. Kondensator nach Anspruch 13,
**dadurch gekennzeichnet,**
**dass** die den Gasabführungskanal (3, 3') umschließende Innenwandung (11, 11') die Kondensationsfläche bildet,
**dass** in einem Abstand zur Innenwandung (11, 11') eine Außenwandung (12) angeordnet ist und
**dass** durch den Zwischenraum (17) zwischen Außenwandung (12) und Innenwandung (11, 11') eine Kühlflüssigkeit durchleitbar ist.

16. Kondensator nach Anspruch 13 oder 15,
**dadurch gekennzeichnet,**
**dass** das Turbulenzerzeugungsmittel (9, 9') als eine Packung (10) aus einem Vliesmaterial oder aus Rohrabschnitten aus Glas, Keramik, Kunststoff oder Metall ausgebildet ist.

## Claims

1. A bioreactor (1) having a container (2) with at least one gas discharge channel (3, 3') for discharging gas, wherein the opening (8) of the gas discharge channel (3, 3') is coupled to a hydrophobic sterile filter (5, 5') and a condenser (4, 4') with condensation surfaces arranged therebetween,
**characterized in**
**that** turbulence generating means (9, 9') generating a turbulent stream are arranged in the region of the condenser (4, 4') in the gas discharge channel (3, 3'), and
**that** the container (2), the condenser (4, 4') and the sterile filter (5, 5') are formed of gamma sterilizable plastic.

2. The bioreactor according to claim 1,
**characterized in**
**that** the external surfaces of the turbulence generating means (9, 9') are configured as condensation surfaces.

3. The bioreactor according to claim 2,
**characterized in**
**that** the turbulence generating means (9, 9') are configured as a condenser hose (20) or condenser tube, arranged as a spiral in the gas discharge channel (3, 3'), through which a coolant can be conducted.

4. The bioreactor according to claim 3,
**characterized in**
**that** the condenser hose (20) or the condenser tube is surrounded by further combined coolants and turbulence generating means.

5. The bioreactor according to claim 1,
**characterized in**
**that** the internal wall (11, 11') enclosing the gas discharge channel (3, 3') forms the condensation surface.

6. The bioreactor according to claim 5,
**characterized in**
**that** an external wall (12) is arranged at a distance to the internal wall (11, 11') and
**that** a coolant can be conducted through the intermediate space (17) between the external wall (12) and the internal wall (11, 11').

7. The bioreactor according to any of claims 1, 5 or 6,
**characterized in**
**that** the turbulence generation means (9, 9') is configured as a package (10).

8. The bioreactor according to claim 7,
**characterized in**
**that** the package (10) is formed from a fibrous non-woven web material.

9. The bioreactor according to claim 7,
**characterized in**
**that** the package (10) is formed from tube sections made of glass, ceramic, plastic or metal.

10. The bioreactor according to any of claims 1 to 9,
**characterized in**
**that** the sterile filter (5, 5') has a heater (24) at its circumference.

11. The bioreactor according to any of claims 1 to 10,
**characterized in**
**that** the sterile filter (5, 5') is integrated in the condenser (4, 4').

12. The bioreactor according to any of claims 1 to 11,
**characterized in**
**that** the container (2), the condenser (4, 4') and the sterile filter (5, 5') are configured as a single-use device.

13. A condenser (4, 4') for a bioreactor (1) according to any of claims 1 to 12, having a housing (22, 22') with a gas discharge channel (3, 3') for conducting a gas and condensation on condensation surfaces,
**characterized in**
**that** turbulence generating means (9, 9') generating a turbulent stream are arranged in the region of the condenser (4, 4') in the gas discharge channel (3, 3') having a hydrophobic sterile filter (5, 5'), and the condenser (4, 4') as well as the sterile filter (5, 5') are formed of gamma sterilizable plastic.

14. The condenser according to claim 13,
**characterized in**
**that** the turbulence generation means (9, 9') are configured as a spiral-shaped condenser hose (20) or condenser tube arranged in the gas discharge channel (3, 3'), through which a coolant can be conducted.

15. The condenser according to claim 13,
**characterized in**
**that** the internal wall (11, 11') enclosing the gas discharge channel (3, 3') forms the condensation area, that an external wall (12) is arranged at a distance to the internal wall (11, 11') and
**that** a coolant can be conducted through the intermediate space (17) between the external wall (12) and the internal wall (11, 11').

16. The condenser according to claim 13 or 15,
**characterized in**
**that** the turbulence generation means (9, 9') are formed as a package (10) made of a fibrous non-woven web material or from tube sections made of glass, ceramic, plastic or metal.

## Revendications

1. Bioréacteur (1) avec un contenant (2) avec au moins un canal d'évacuation des gaz (3, 3') destiné à décharger les gaz, dans lequel l'ouverture (8) du canal d'évacuation des gaz (3, 3') est reliée à un filtre stérile (5, 5') hydrophobe et à un condensateur (4, 4') disposé entre ceux-ci avec des surfaces de condensation,
**caractérisé en ce**
**que** des moyens de génération de turbulence (9, 9') générant un écoulement turbulent sont disposés dans la zone du condensateur (4, 4') dans le canal d'évacuation des gaz (3, 3'), et
**que** le contenant (2), le condensateur (4, 4') et le filtre stérile (5, 5') sont réalisés à partir de matière plastique pouvant être stérilisée par irradiation gamma.

2. Bioréacteur, selon la revendication 1,
**caractérisé en ce**
**que** les surfaces extérieures des moyens de génération de turbulence (9, 9') sont réalisées sous la forme de surfaces de condensation.

3. Bioréacteur selon la revendication 2,
**caractérisé en ce**
**que** le moyen de génération de turbulence (9, 9') est réalisé sous la forme d'un tuyau de condensateur (20) ou tube de condensateur, disposé de manière hélicoïdale dans le canal d'évacuation des gaz (3, 3'), par lequel un liquide de refroidissement peut être guidé.

4. Bioréacteur selon la revendication 3,
**caractérisé en ce**
**que** le tuyau de condensateur (20) ou le tube de condensateur sont entourés par d'autres moyens de génération de turbulence et de refroidissement combinés.

5. Bioréacteur selon la revendication 1,
**caractérisé en ce**
**que** la paroi intérieure (11, 11') entourant le canal d'évacuation des gaz (3, 3') forme la surface de condensation.

6. Bioréacteur selon la revendication 5,
**caractérisé en ce**
**qu'**une paroi extérieure (12) est disposée à une certaine distance de la paroi intérieure (11, 11') et
**qu'**un liquide de refroidissement peut être amené à passer à travers l'espace intermédiaire (17) entre la paroi extérieure (12) et la paroi intérieure (11, 11').

7. Bioréacteur selon l'une quelconque des revendications 1, 5 ou 6,
**caractérisé en ce**
**que** le moyen de génération de turbulence (9, 9') est réalisé sous la forme d'un garnissage (10).

8. Bioréacteur selon la revendication 7,
**caractérisé en ce**
**que** le garnissage (10) est réalisé à partir d'un matériau non tissé.

9. Bioréacteur selon la revendication 7,
**caractérisé en ce**
**que** le garnissage (10) est réalisé à partir de segments tubulaires en verre, céramique, matière plastique ou métal.

10. Bioréacteur selon l'une quelconque des revendications 1 à 9,
**caractérisé en ce**
**que** le filtre stérile (5, 5') présente sur sa périphérie un dispositif de chauffage (24).

11. Bioréacteur selon l'une quelconque des revendications 1 à 10,
**caractérisé en ce**
**que** le filtre stérile (5, 5') est intégré dans le condensateur (4, 4').

12. Bioréacteur selon l'une quelconque des revendications 1 à 11,
**caractérisé en ce**
**que** le contenant (2), le condensateur (4, 4') et le filtre stérile (5, 5') sont réalisés sous la forme d'un dispositif jetable.

13. Condensateur (4, 4') pour un bioréacteur (1) selon l'une quelconque des revendications 1 à 12, avec un boîtier (22, 22') avec un canal d'évacuation des gaz (3, 3') pour le passage d'un gaz et la condensation sur des surfaces de condensat,
**caractérisé en ce**
**que** des moyens de génération de turbulence (9, 9') générant un écoulement turbulent sont disposés dans la zone du condensateur (4, 4') dans le canal d'évacuation des gaz (3, 3') avec le filtre stérile (5, 5') hydrophobe et le condensateur (4, 4') ainsi que le filtre stérile (5, 5') sont réalisés à partir de matière plastique pouvant être stérilisée par irradiation gamma.

14. Condensateur selon la revendication 13,
**caractérisé en ce**
**que** le moyen de génération de turbulence (9, 9') est réalisé sous la forme d'un tuyau de condensateur (20) ou tube de condensateur disposé de manière hélicoïdale dans le canal d'évacuation des gaz (3, 3'), par lesquels un liquide de refroidissement peut être guidé.

15. Condensateur selon la revendication 13,
**caractérisé en ce**
**que** la paroi intérieure (11, 11') entourant le canal d'évacuation des gaz (3, 3') forme la surface de condensation,
**qu'**une paroi extérieure (12) est disposée à une certaine distance de la paroi intérieure (11, 11') et
**qu'**un liquide de refroidissement peut être amené à passer à travers l'espace intermédiaire (17) entre la paroi extérieure (12) et la paroi intérieure (11, 11').

16. Condensateur selon la revendication 13 ou 15,
**caractérisé en ce**
**que** le moyen de génération de turbulence (9, 9') est réalisé sous la forme d'un garnissage (10) composé d'un matériau non tissé ou de segments tubulaires en verre, céramique, matière plastique ou métal.
